Europäisches Patentamt
European Patent Office
Office européen des brevets

(11) Publication number: 0 358 085 A1

# EUROPEAN PATENT APPLICATION

(21) Application number: 89115883.4

(22) Date of filing: 29.08.89

(51) Int. Cl.[5]: C07D 477/00 , A61K 31/40

Claims for the following Contracting States: ES + GR.

(30) Priority: 29.08.88 JP 212441/88

(43) Date of publication of application: 14.03.90 Bulletin 90/11

(84) Designated Contracting States: AT BE CH DE ES FR GB GR IT LI LU NL SE

(71) Applicant: LEDERLE (JAPAN) LTD.
Hattori Bldg., 5th Floor 10-3 Kyobashi
1-chome
Chuo-ku Tokyo(JP)

(72) Inventor: Kumagai, Toshio
3-15-37, Kosenba-cho
Kawagoe-shi Saitama-ken(JP)
Inventor: Tamai, Satoshi
2-21-3, Motoizumi
Komae-shi Tokyo(JP)
Inventor: Hayashi, Takaaki
5-8-10, Nakamuneoka
Shiki-shi Saitama-ken(JP)

(74) Representative: Kraus, Walter, Dr. et al
Patentanwälte Kraus, Weisert & Partner
Thomas-Wimmer-Ring 15
D-8000 München 22(DE)

## (54) (1R,5S,6S)-6-[(R)-1-Hydroxyethyl]-1-methyl-2-[(1-alkyl-1,2,4-triazolium-2-yl)alkyl]-thio-carbapenem-3-carboxylic acid derivatives.

(57) Novel carbapenem compounds, (1R,5S,6S)-6-[(R)-1-hydroxyethyl]-1-methyl-2-[(1-alkyl-1,2,4-triazolium-2-yl)-alkyl]thio-carbapenem-3-carboxylic acid derivatives. These carbapenem compounds are represented by the following formula having a beta-coordinated methyl group introduced into the 1-position and a 1-alkyl-1,2,4-triazolium-2-yl alkylthio group introduced into the 2-position.

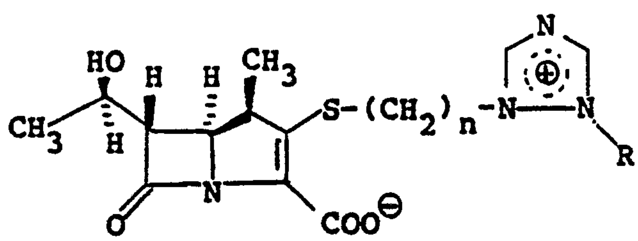

wherein R is a lower alkyl group and n is 1, 2 or 3.
They have antibacterial activity, and are used for prevention and treatment of bacterial infections as antibacterial agents.

EP 0 358 085 A1

## BACKGROUND OF THE INVENTION

1. Field of the Invention

The present invention relates to a carbapenem antibiotic and, more particularly, to a 1β-methyl-carbapenem derivative having a methyl group introduced at the 1-position of the carbapenem skeleton to an antibacterial composition containing the same as an active ingredient, and to a process of preparing the same.

2. Description of the Prior Art

Heretofore, as various antibacterial substances, there have been proposed many carbapenem antibiotic substances having, as a basic skeleton, carba-2-penem-3-carboxylic acid represented by the following formula (A):

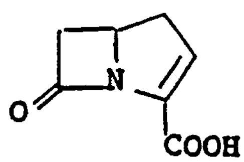

(A)

For example, an initial generation of carbapenem antibiotics is a naturally occurring carbapenem compound such as thienamycin represented by the formula (B):

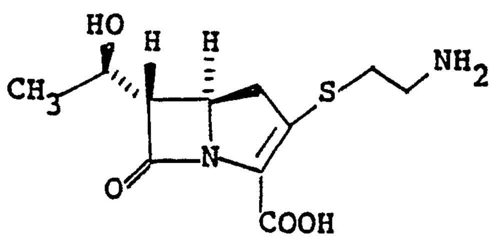

(B)

The thienamycin may be obtained from a fermentation broth of Streptomyces cattleya and has a broad range of antibacterial spectra against Gram-positive and Gram-negative bacteria. It has been expected therefore to be developed as a highly useful compound, but its poor chemical stability has precluded its commercialization.

With the foregoing background, many researchers have attempted to develop a carbapenem compound having antibacterial activities as high as thienamycin and ensuring more chemical stability. As a result, there has been developed imipenem (INN) represented by the following formula (C):

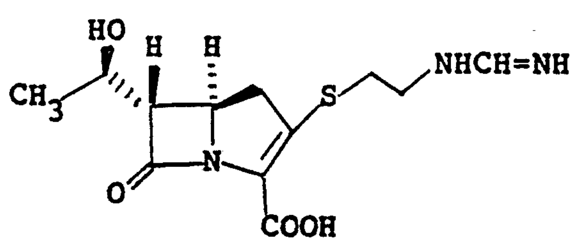

(C)

This compound is a practically available antibacterial agent and may be obtained by converting an amino group as a side chain at the 2-position to a formimidoyl group.

The imipenem of the formula (C) exhibits antibacterial activities higher than those of the thienamycin and ensures some degree of chemical stability; however, it presents the disadvantage that it is decomposd

within a short period of time by kidney dehydropeptidase (DHP) in the living body. For this reason, it cannot be administered singly, and must be used in combination with a DHP inhibitor in order to control its decomposition leading to inactivation. Its formulation for clinical administration is a combination with cilastatin (INN) that is a DHP inhibitor.

An antibacterial agent preferred for practical clinical use, however, is one that alone can demonstrate antibacterial activity. Furthermore, the DHP inhibitor to be combined with the antibiotic could exert undesirable actions on tissues of the living body. For these reasons, the combined use should be avoided wherever possible. Thus there has been a growing demand for a carbapenem compound having sufficiently high degrees of both antibacterial activity and resistance to DHP.

Recently, there were proposed some carbapenem compounds of the type that could achieve the above objectives. Such carbapenem compounds are 1-methylcarbapenem compounds in which a methyl group is introduced at the 1-position of the carbapenem skeleton. Most recently, another type of carbapenem compounds was proposed which has a heterocycloalkylthio group at the 2-position of the carbapenem skeleton. For example, European Patent Publication No. 168,707 (Japanese Laid-Open Patent Publication No. 636,791/1986) to Merck discloses 1-methylcarbapenem compounds having at the 2-position an alkylated mono- or bi-cyclic quaternary heteroaryl alkylthio substituent, represented by the formula (D):

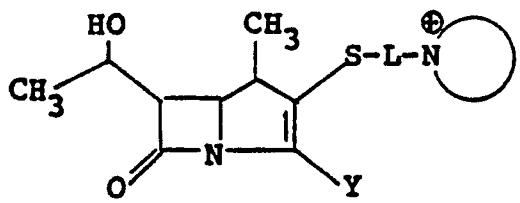
(D)

It is reported that these compounds have superior antibacterial activities as well as a remarkably improved resistance to decomposition by DHP leading to inactivation so that they demonstrate highly useful effects.

It should be noted here that the Japanese Laid-Open Patent Publication No. 636,791/1986 discloses merely a general concept of 1$\beta$-methyl-carbapenem compounds and a very limited number of specific working examples. It also discloses in generic terms that they are superior in antibacterial activities; however, it does not specifically describe any antibacterial data whatsoever. Furthermore, the Japanese patent document merely enumerates more than about 300 compounds by their names only; but it contains only 16 compounds that have been actually supported by working examples. No specific compounds according to the present invention are disclosed therein and the prior patent application quoted above does not hint anything about such compounds having superior pharmacological characteristics as demonstrated and claimed by the present invention.

## SUMMARY OF THE INVENTION

The present invention provides carbapenem compounds having high antibacterial activities, a strong action of inhibiting $\beta$-lactamase as well as improved resistance to kidney dehydropeptidase. More specifically, the present invention provides the carbapenem compounds substituted by a methyl group at the 1-position in the $\beta$-configuration, in which particularly a triazolium-2-yl alkyl thio group is introduced at the 2-position.

Specifically, the present invention provides (1R,5S,6S)-6-[(R)-1-hydroxyethyl]-1-methyl-2-[(1-alkyl-1,2,4-triazolium-2-yl)alkyl]thio-carbapenem-3-carboxylate represented by formula (I):

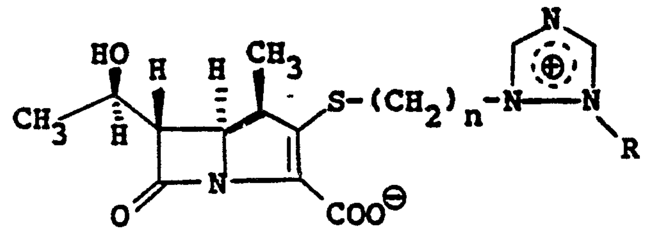
(I)

wherein R is a lower alkyl group, and n is 1, 2 or 3,

or a pharmaceutically acceptable salt thereof.

The present invention further provides an antibacterial composition containing the carbapenem compound represented by formula (I) or a pharmaceutically acceptable salt thereof, as an active ingredient.

For the above purpose of the invention, preferred carbapenem compound of formula (I) is (1R,5S,6S)-6-[(R)-1-hydroxyethyl]-1-methyl-2-[2-(1-methyl-1,2,4-triazolium-2-yl)ethyl)thio-carbapenem-3-carboxylate as formula (I-1):

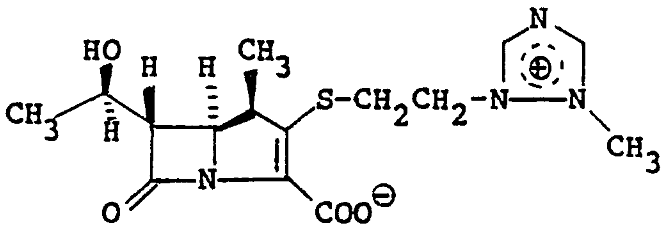

(I-1)

or a pharmaceutically acceptable salt thereof.

In this specification and claims, a triazolium-2-yl group introduced in the side chain at 2-position of the above formulas (I) and (I-1) represents following:

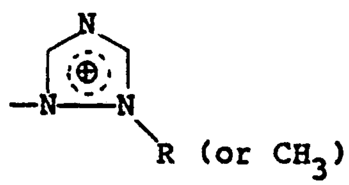

However, this formula is thought to take a tautomeric form, that is a formula (1) or (2) as shown below because of the presence of conjugated form at the quaternary ammonium nitrogen atoms.

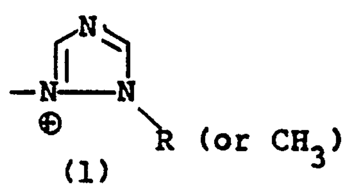

(1)

or

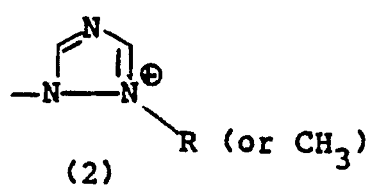

(2)

Therefore, triazolium-2-yl group is described as above formula throughout this specification and claims.

Furthermore, the present invention provides a crystalline carbapenem compound of the formula (I-1) or a pharmaceutically acceptable salt thereof.

## DETAILED DESCRIPTION OF THE INVENTION

The carbapenem compounds according to the present invention are novel compounds that are not specifically disclosed in the prior patent publications (for instance, Japanese Patent Laid-Open Publication No. 636,791/1986). In particular, they are remarkably characterized in that the substituent at the 2-position of the carbapenem skeleton is a 1,2,4-triazolium-2-yl alkylthio group and in that they have superior antibacterial activities and resistance to DHP.

In accordance with the present invention, the carbapenem compounds represented by formula (I) may be prepared basically by a process to be described below.

Briefly stated, the carbapenem of formula (I) may be prepared by reacting a compound represented by formula (II):

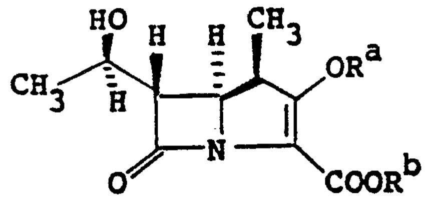

(II)

wherein $R^a$ is an acyl group, and $R^b$ is a carboxyl protecting group, with a mercapto reagent represented by formula (III):

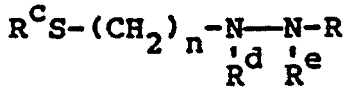

(III)

wherein $R^c$ is a hydrogen atom or an acyl group;
$R^d$ and $R^e$ are amino protecting groups; and R and n have the same meanings as above, to give a compound represented by formula (IV):

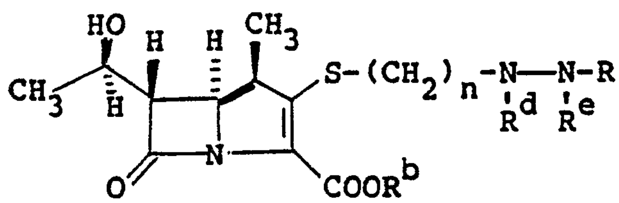

(IV)

wherein R, $R^b$, $R^d$, $R^e$ and n have the same meanings as above, and subjecting the compound of the formula (IV) to removal of the amino protecting groups $R^d$ and $R^e$, and of the carboxyl protecting group $R^b$ respectively, and then reacting with forminidic acid ester to give the carbapenem compound represented by formula (I)

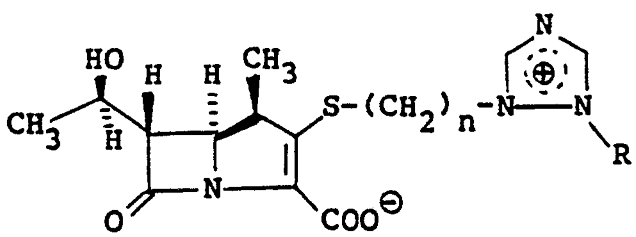

(I)

wherein R and n have the same meanings as above.

More specifically, the carbapenem compounds represented by formula (I) may be prepared in such a manner as will be described in detail below.

In the specification of the present application, the term "lower" qualifying a group of a compound means that the group or compound so qualified has from 1 to 7, preferably from 1 to 4, carbon atoms.

The term "lower alkyl" referred to herein stands for a straight-chained or branched-chain hydrocarbon group having preferably from 1 to 6 carbon atoms and may include, for example, methyl, ethyl, n-propyl, isopropyl, n-butyl, isobutyl, sec-butyl, tert.-butyl, n-pentyl, isopentyl, n-hexyl, isohexyl or the like.

The term "carboxyl protecting group" referred to herein stands for any group capable of protecting the carboxyl group of the compound involved without adversely affecting any other substituents and the reactions that follow and may include, for example, an ester residue such as a lower alkyl ester residue including, for example, methyl ester, ethyl ester, n-propyl ester, isopropyl ester, n-, iso-, sec- or tert.-butyl ester, n-hexyl ester or the like; an aralkyl ester residue including, for example, benzyl ester, p-nitrobenzyl ester, o-nitrobenzyl ester, p-methoxybenzyl ester or the like; and a lower aliphatic acyloxymethyl ester residue including, for example, acetoxymethyl ester, propionyloxymethyl ester, n- or iso-butyryloxymethyl ester, pivaloyloxymethyl ester or the like.

The term "acyl group" referred to herein stands for, in a narrower sense, a moiety obtainable by removing the hydroxyl group from the carboxyl group of an organic carboxylic acid as well as, in a broader sense, any acyl group derived from an organic sulfonic acid or an organic phosphoric acid. Such an acyl group may include, for example, a lower alkanoyl group such as acetyl, propionyl, butyryl or the like, a (halo)lower alkyl sulfonyl group such as methanesulfonyl, trifluoromethanesulfonyl or the like; a substituted or unsubstituted arylsulfonyl group such as benzenesulfonyl, p-nitrobenzenesulfonyl, p-bromobenzenesulfonyl, toluenesulfonyl, 2,4,6-triisopropylbenzenesulfonyl or the like; and diphenylphosphoryl.

The term "amino protecting group" referred to herein stands for groups usually employed in peptide chemistry, for example, aromatic acyl groups such as phthaloyl, benzoyl, benzoyl substituted by halogen, nitro or a lower alkyl of 1 to 4 carbon atoms (e.g. chlcrobenzoyl, p-nitrobenzoyl, p-tert-butylbenzoyl, toluoyl), naphthoyl; phenylacetyl; phenoxyacetyl; benzenesulfonyl; benzenesulfonyl substituted by a lower alkyl of 1 to 4 carbon atoms (e.g. p-tert-butylbenzenesulfonyl, toluenesulfonyl); acyl derived from aliphatic or halogenated aliphatic carboxylic acid such as acetyl, valeryl, caprylyl, n-decanoyl, acyloyl, pivaloyl, halogenoacetyl (e.g. monochloroacetyl, monobromoacetyl, dichloroacetyl, trichloroacetyl); camphorsulfonyl; methanesulfonyl; esterified carboxyl groups such as ethoxycarbonyl, tert-butyloxycarbonyl, isobornyloxycarbonyl, phenyloxycarbonyl, trichloroethoxycarbonyl, benzyloxycarbonyl, etc.; carbamoyl groups such as methylcarbamoyl, phenylcarbamoyl, naphthylcarbamoyl, etc.; and the corresponding thiocarbamoyl groups.

The compound represented by formula (II) is reacted with a mercapto reagent represented by formula (III) to give the compound represented by formula (IV).

The reaction of the compound of formula (II) with the mercapto reagent of formula (III) may be carried out, for instance, by reacting the compound of formula (II) with the mercapto reagent of formula (III) in an excess amount ranging from about an equimolar amount to approximately 1.5 molar amount in an appropriate solvent such as tetrahydrofuran, dichloromethane, dioxane, dimethylformamide, dimethylsulfoxide; acetonitrile, hexamethylene phosphoramide or the like, preferably in the presence of a base such as sodium hydrogen carbonate, potassium carbonate, triethylamine, diisopropylethyl amine or the like at a temperature range from approximately -40 °C to approximately 25 °C for approximately 30 minutes to approximately 24 hours.

In the above reaction, when the mercapto reagent of formula (III) in which the acyl group $R^c$ is used, this mercapto reagent is converted to a corresponding free thiol derivative by reacting with a base such as methanol alkoxide, for example sodium methoxide or sodium ethoxide; or ammonia and then the resultant free thiol derivative is reacted with the compound of formula (II) to give the compound represented by formula (IV).

The reaction described above provides the carbapenem compound represented by formula (IV) in which the carboxyl group at the 3-position thereof is protected by the carboxyl protecting group $R^b$ and the substituent at 2-position thereof is protected by the amino protecting groups $R^d$ and $R^e$. The removal of the protecting groups $R^b$, $R^d$ and $R^e$ may be made by a reaction known per se for removing a protective group, such as solvolysis or hydrogenolysis. In a typical reaction, the compound represented by formula (IV) may be treated, for instance, in a mixture of solvents such as tetrahydrofuran-water, tetrahydrofuran-ethanol-water, dioxane-water, dioxane-ethanol-water, n-butanol-water or the like containing morpholino-propane sulfonic acid-sodium hydroxide buffer solution (pH 7), a phosphate buffer solution (pH 7), dipotassium phosphate, sodium bicarbonate or the like, using hydrogen under 1 to 4 atmospheric pressures, in the presence of a catalyst for hydrogenation such as platinum oxide, palladium-activated carbon or palladium hydroxide-activated carbon at temperatures ranging from approximately 0 °C to approximately 50 °C for approximately 0.25 to approximately 4 hours.

As a result, the compound represented by the following formula (V):

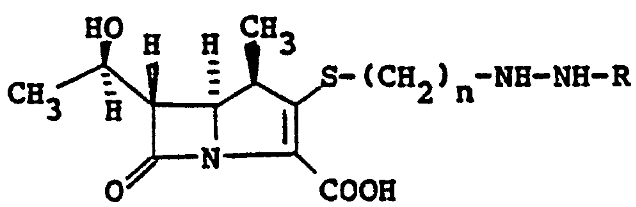

(V)

wherein R and n have the same meanings as above,
is provided.

Then, this compound of formula (V) is converted to (1R,5S,6S)-6-[(R)-1-hydroxyethyl]-1-methyl-2-[(1-alkyl-1,2,4-triazolium-2-yl)alkyl]thio-carbapenem-3-carboxylate of the formula (I) by reacting with a formimidic acid ester derivative. The reaction may be carried out by reacting the compound of formula (V) with a formimidic acid ester derivative such as ethyl formimidate hydrochloride, methyl formimidate hydrochloride or benzyl formimidate hydrochloride in the presence of a base such as sodium bicarbonate, potassium carbonate or triethylamine under neutrated buffer conditions (for example, morpholinopropanesulfonic acid-sodium hydroxide buffer having a pH of 7.0 or phosphate buffer having a pH of 7.0) at temperatures ranging from approximately -10° C to approximately 80° C, preferably from approximately 0° C to room temperature.

Thus the objective carbapenem compound of the present invention (1R,5S,6S)-6-[(R)-1-hydroxyethyl]-1-methyl-2-[(1-alkyl-1,2,4-triazolium-2-yl)alkyl]thio-carbapenem-3-carboxylate of the formula (I) can be obtained in good yield and if desired, this compound may be obtained in highly purity one by using an appropriate ion exchange resin such as, preferably, Dowex 50W-X4 type ion exchange resin or polymeric resin such as, preferably, XAD-II type resin, HP-20 type resin or MP-40 type resin.

In actual application, the (1R,5S,6S)-6-[(R)-1-hydroxyethyl]-1-methyl-2-[(1-alkyl-1,2,4-triazolium-2-yl)-alkyl]thio-carbapenem-3-carboxylate of formula (I) may be converted to a preferred crystalline compound. This crystallization may be carried out by treating the compound of formula (I) preferably in water or in an aqueous medium such as water-alcohol or water-alcohol-acetone, and the resulting crystals may be in the form of an anhydrous compound or a hydrate.

The compound of the formula (II) as using for the starting compound in the above reaction may be prepared by a reaction between a compound represented by the formula (VI):

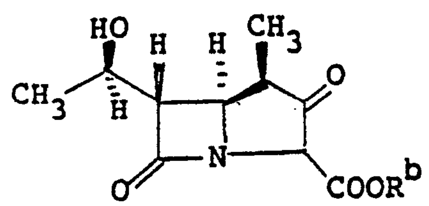

(VI)

wherein $R^b$ has the same meaning as above,
and a reactive derivative of an acid represented by the formula (VII)

$R^aOH$ (VII)

wherein $R^a$ has the same meaning as above.

The reactive acid derivative may include, for example, an acid anhydride such as acetic acid anhydride, methanesulfonic acid anhydride, p-toluenesulfonic acid anhydride, p-nitrobenzenesulfonic acid anhydride, 2,4,6-triisopropylbenzenesulfonic acid anhydride, trifluoromethanesulfonic acid anhydride or the like or an acid halide such as an acid chloride, i.e., acetyl chloride, propionyl chloride, diphenylphosphoric chloride, toluenesulfonyl chloride, p-bromobenzenesulfonyl chloride or the like. Diphenylphosphoric chloride ($R^a$ = diphenylphosphoryl group) is particularly preferred.

The reaction of the compound of formula (VI) with the reactive acid derivative may be carried out, for example, in a manner similar to a conventional acylation reaction in an inert solvent such as methylene chloride, acetonitrile, dimethylformamide or the like, conveniently in the presence of a base such as diisopropylethylamine, triethylamine, 4-dimethylaminopyridine or the like at temperatures ranging from -20° C to 40° C for approximately 30 minutes to approximately 24 hours.

The compound represented by formula (VI) to be employed as a starting compound in the process described above is known per se and may be prepared in such a manner as disclosed, for example, in U. S. Patent 4,312,871 (Japanese Laid-Open Patent Publication No. 123,985/1981) or, more preferably, in accordance with the spatially selective method as disclosed, for example, in Japanese Laid-Open Patent Publication No. 170,377/1988).

On the other hand, the mercapto reagent represented by formula (III) may include compounds represented below:

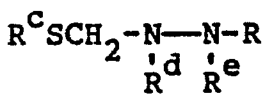 (III-1)

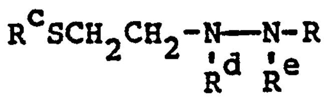 (III-2)

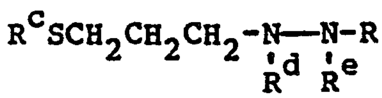 (III-3)

wherein R, $R^c$, $R^d$ and $R^e$ have the same meanings as above,
for example,

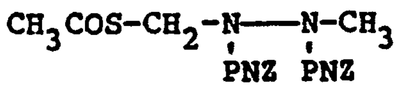 (III-1-1)

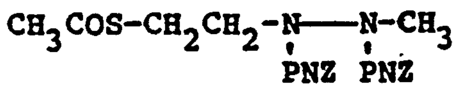 (III-2-1)

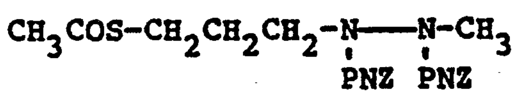 (III-3-1)

wherein PNZ is p-nitrobenzyloxycarbonyl group.

The mercapto reagent represented by formula (III-2-1) can be obtained by the way described in Example 1 or 2. The other mercapto reagents can also be produced in a similar manner.

The desired compounds of formula (I) in accordance with the present invention are novel compounds that are not disclosed specifically in the above-mentioned publication and that are extremely stable against dehydropeptidase (DHP) known as a kidney enzyme and have superior antibacterial activities. The remarkably high antibacterial activities and stability against the kidney DHP of the compounds of formula (I) according to the present invention have been determined by biological tests described below.

I. Antibacterial Tests

Test Procedures:

The antibacterial activities were tested by an agar plate dilution method in accordance with the standard method of The Japanese Chemotherapy Society [Chemotherapy, Vol. 29, 76-79 (1981)].

A Mueller-Hinton (MH) agar liquid medium of a test microorganism was cultured overnight at 37° C and the resultant culture medium was diluted with a buffered saline gelatin (BSG) solution to contain approximately $10^6$ cells of the test microorganisms per milliliter, and then the diluted solution was inoculated with a micro-planter at the rate of approximately 5 microliters on a MH agar medium containing a test compound. This medium was then incubated at 37° C for 18 hours. The minimum inhibitory concentration (MIC) is determined as a minimum concentration in which no test microorganism could grow. It is noted here that the test organisms used were all standard strains.

Results:

Table 1 shows the test results. The test compound used therein was the compound (11) obtained in

Example 3. As a control compound, imipenem was used as a carbapenem compound which is clinically employed.

Table 1

| MINIMUM INHIBITORY CONCENTRATIONS (MIC) | | |
|---|---|---|
| | MIC (μg/ml) | |
| | Test Compounds | |
| Test Organisms | Imipenem | Compd. (11) |
| Staphylococcus aureus FDA 209PJC-1 | 0.013 | 0.1 |
| Staphylococcus aureus Terajima | <0.006 | 0.025 |
| Staphylococcus aureus MS353 | 0.013 | 0.05 |
| Streptococcus pyogenes Cook | <0.006 | 0.05 |
| Micrococcus luteus ATCC9341 | <0.006 | 0.1 |
| Bacillus subtilis ATCC6633 | 0.025 | 0.2 |
| Escherichia coli NIHJ JC-2 | 0.1 | 0.1 |
| Escherichia coli K12 C600 | 3.13 | 0.78 |
| Enterobacter aerogenes ATCC1304 | 1.56 | 0.78 |
| Enterobacter cloacae 963 | 0.78 | 0.1 |
| Klebsiella pneumoniae PCI-602 | 0.2 | 0.2 |
| Salmonella typhimurium IID971 | 0.2 | 0.2 |
| Salmonella typhi 901 | 0.05 | 0.05 |
| Salmonella paratyphi 1015 | 1.56 | 0.78 |
| Salmonella schottmuelleri 8006 | 0.78 | 0.39 |
| Salmonella enteritidis G14 | 1.56 | 0.78 |
| Serratia marcescens IAM1184 | 0.2 | 0.2 |
| Morganella morganii IFO3848 | 3.13 | 0.39 |
| Proteus mirabilis IFO3849 | 6.25 | 1.56 |
| Proteus vulgaris OX-19 | 3.13 | 0.78 |
| Proteus vulgaris HX-19 | 3.13 | 0.78 |
| Providencia rettgeri IFO 3850 | 0.05 | 0.05 |
| Pseudomonas aeruginosa IFO 3445 | 0.78 | 1.56 |
| Pseudomonas aeruginosa NCTC 10490 | 0.39 | 0.78 |

The foregoing results clearly demonstrate that the compound (11) according to the present invention have superior antibacterial activities.

II. Stability Test against Kidney Dehydropeptidase:

1.Materials:

(1) Swine Kidney Dehydropeptidase-I (DHP-I):

The swine kidney (8 kg) was homogenized and the enzyme protein was allowed to precipitate. After a connective lipid was removed with acetone, the resultant material was made soluble by treatment with butanol and purified by the ammonium sulfate fraction method, thereby producing DHP-I enzyme from a 75 % ammonium sulfate fraction.

The DHP-I enzyme was then adjusted to give an enzyme concentration of 25 mg/10 ml (phosphate buffer, pH 7.1), and divided into 1 ml portions. The portions were frozen and stored at -40°C or less until use.

(2) Test Compound.

Compound (11) obtained in Example 3 below was used as a test compound.

The test compound was adjusted in situ to give a concentration of 117µM with a 50 mM sodium phosphate buffer solution (pH =7.1).

Glycyl dehydrophenylalanine (Gl-dh-Ph) and imipenem were employed as control compounds, and they were adjusted in situ each to give a concentration of 117µM with the same sodium phosphate buffer solution.

2.Method:

(1) Measurement for Hydrolysis Activity against DHP-I Enzyme Substrate by Late Assay:

To 1.2 ml of 50 mM sodium phosphate buffer solution (substrate) containing 117µM of each of Gl-dh-Ph and imipenem as the control compounds was added 0.2 ml of the DHP-I enzyme solution (25 mg/10 ml) prepared above in the final substrate concentration of 100µM. The solution was then incubated at 37° C for 10 minutes. The initial velocity of hydrolysis of the substrate was measured from a decrease in absorbency at a particular λmax of each of the substrates.

A blank test was conducted in substantially the same manner as above by adding 0.2 ml of the sodium phosphate buffer solution (pH 7.1) to 1.2 ml of the above substrate.

(2) Measurement for Stability of Test Compounds against DHP-I by High Performance Liquid Chromatography Method (HPLC):

The test compound according to the present invention and the control compounds were treated in substantially the same manner as (1) above. The incubation, however, was conducted at 37° C for 4.5 hours or for 24 hours. The degree of hydrolysis of the compounds each after the test periods was measured by the HPLC method.

3.Results:

The initial velocity of hydrolysis of each of the substrates against DHP-I by the late assay was found as follows:

Gl-dh-Ph: 17.4.µM/minute

Imipenem: 0.56 µM/minute

Table 2 below shows measurement results on stability of the compound according to the present invention and imipenem against DHP-I.

Table 2

| DEGREES OF HYDROLYSIS BY DHP-I | | |
|---|---|---|
| (Method: HPLC; Substrate Concentration: 100 µM; Unit: µM) | | |
| | Test Compounds | |
| Incubation Conditions | Imipenem | Compound (11) |
| 37° C, 4.5 hours | 77.6 | 2.6 |
| 37° C, 24 hours | * | 2.8 |

* After 24 hours at 37° C, it has been found that most or all imipenem has been decomposed and nothing remained was detected.

The stability test results against DHP-I clearly show that the carbapenem compound according to the present invention was approximately thirty times as stable imipenem.

III.Toxicity:

Toxicological studies were carried out using a group of 10 male mice of CrjCD(SD) strain weighing from 20 to 23 grams. A solution containing the carbapenem compound (11) of the present invention obtained by Example 3 was administered subcutaneously to the mice and subjected to observations for one week.

The results have revealed that the group of mice to which the carbapenem compound (11) of the present invention had been administered in the amount of 500 mg/kg were alive without any abnormal observations.

As described above, the carbapenem compounds according to the present invention demonstrate remarkable antibacterial activities comparable to imipenem as well as an overwhelmingly higher resistance against DHP than imipenem.

Therefore, the carbapenem compounds of formula (I) according to the present invention permit a single administration without combination with any other compounds and without a risk of any side effect that might be caused in their combined use with a DHP inhibitor, unlike imipenem that was led for the first time to a practically useful antibacterial agent in combination with cilastatin acting as a DHP inhibitor. The carbapenem compounds are accordingly extremely useful as antibacterial agents for therapy and prevention of infectious diseases from various pathogenic organisms.

The carbapenem compound of formula (I) according to the present invention may be administered as an antibacterial agent to the human being and other mammalian animals in the form of a pharmaceutically acceptable composition containing an antibacterially effective amount thereof. The administration dose may vary in a wide range with ages, patients, weights and conditions of patients, forms or routes of administration, physicians' diagnoses or the like and may be orally, parenterally or topically administered, to adult patients usually in a standard daily dose range from approximately 200 to approximately 3,000 mg once or in several installments per day.

The pharmaceutically acceptable composition of the carbapenem compound of formula (I) according to the present invention may contain an inorganic or organic, solid or liquid carrier or diluent, which is conventionally used for preparations of medicines, particularly antibiotic preparations, such as an excipient, e.g., starch, lactose, white sugar, crystalline cellulose, calcium hydrogen phosphate or the like; a binder, e.g., acacia, hydroxypropyl cellulose, alginic acid, gelatin, polyvinyl pyrrolidone or the like; a lubricant, e.g., stearic acid, magnesium stearate, calcium stearate, talc, hydrogenated plant oil or the like; a disintegrator, e.g., modified starch, calcium carboxymethyl cellulose, low substituted hydroxypropyl cellulose or the like; or a dissolution aid, e.g., a non-ionic surface active agent, an anionic surface active agent or the like, and may be prepared into forms suitable for oral, parenteral or topical administration. The formulations for oral administration may include solid preparations such as tablets, coatings, capsules, troches, powders, fine powders, granules, dry syrups or the like or liquid preparations such as syrups or the like; the formulations for parenteral administration may include, for example, injectable solutions, drip-feed solutions, depositories or the like; and the formulations for topical administra tion may include, for example, ointments, tinctures, creams, gels or the like. These formulations may be formed by procedures known per se to those skilled in the art in the field of pharmaceutical formulations.

The carbapenem compounds of formula (I) according to the present invention are suitably administered in the form of parenteral formulations, particularly in the form of injectable solutions.

The production of the carbapenem compounds of the formula (I) according to the present invention will be described more in detail by way of working examples.

In the following description, the following symbols are used to have the particular meanings.

ph : phenyl group
PNB : p-nitrobenzyl group
PNZ : p-nitrobenzyloxycarbonyl group
$\geq$Si : trimethylsilyl group

Example 1:

$CH_3COS-CH_2CH_2-N—N-CH_3$ Compound (1)

PNZ PNZ

(a)

$H_2NNHCH_3 \longrightarrow PNZ-NH-N-CH_3$ Compound (2)

PNZ

To a solution of 4.3 g of p-nitrobenzylchloroformate in 30 ml of dioxane were added 1.66 g of sodium bicarbonate, 3 ml of water and 0.53 mg (10 ml) of methylhydrazine under ice-cooling, and the reaction mixture was stirred for 30 minutes at the same temperature and then for 1 hour at room temperature. After reaction, ethyl acetate was added to the reaction mixture, and the organic layer was washed with water and saturated sodium chloride solution. The resulting solution was dried over magnesium sulfate and the solvent was removed under reduced pressure to give the Compound (2) as pale yellow oil.

MNR ($CDCl_3$) $\delta$ : 3.27 (3H, s), 5.27 (4H, s), 6.90 (1H, bs), 7.50 (4H, d, J = 8.0Hz), 8.20 (4H, d, J = 8.0Hz).

(b)

Compound (2) $\longrightarrow$ $CH_3OCOCH_2-N—N-CH_3$

PNZ PNZ

Compound (3)

1.616 g of the Compound (2) obtained in the step (a) was dissolved in 20 ml of anhydrous tetrahydrofuran, and to this solution was added 160 mg of sodium hydride (60 %) under ice-cooling and the reaction mixture was stirred for 30 minutes. Then, 765 mg of methyl bromoacetate was added to this mixture and the stirring was continued for 30 minutes at the same temperature and for 30 minutes at room temperature respectively. After reaction, ethyl acetate was added to the reaction mixture and the organic layer was separated, then washed with water and saturated sodium chloride solution. The resulting solution was dried over magnesium sulfate and the solvent was removed under reduced pressure. The resulting residue was purified by silica gel column chromatography (n-hexane:ethyl acetate = 1:1) to give 973.4 mg (50 %) of Compound (3) as a yellow oil.

MNR ($CDCl_3$) $\delta$ : 3.30 (3H, s), 3.50-4.20 (2H), 3.80 (3H, s), 5.30 (4H, s), 7.30-7.60 (4H, m), 8.00-8.30 (4H, m).

(c)

Compound (3) $\longrightarrow$ $HO-CH_2CH_2-N—N-CH_3$

PNZ PNZ

Compound (4)

To a solution of 950 mg of Compound (3) obtained in the step (b) above in 200 ml of tetrahydrofuran was added 120 mg of sodium borohydride under ice-cooling. After the reaction mixture was stirred for 10 minutes and reached to room temperature, then each of 0.5 ml of methanol was added to the reaction

mixture every one hour and the stirring was continued for 7 hours. After reaction, chloroform was added to the reaction mixture and the organic layer was separated. The resulting solvent was washed with 1N-hydrogen chloride solution and saturated sodium chloride solution, then dried over magnesium sulfate. The solvent was removed under reduced pressure and the resulting residue was purified by silica gel column chromatography (n-hexane:ethyl acetate = 1:1) to give 540 mg (60 %) of Compound (4) as pale yellow oil.
MNR ($CDCl_3$) $\delta$ : 3.25 (3H, s), 3.50-3.90 (4H, m), 5.30 (4H, s), 7.30-7.60 (4H, m), 8.0-8.30 (4H, m).

(d)

Compound (4)

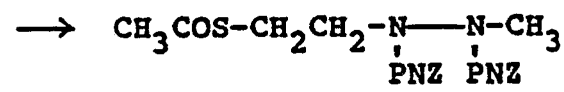

Compound (1)

To an ice-cooling solution of 895 mg of Compound (4) obtained in the step (c) above in 5 ml of dichloromethane were added dropwise 0.16 ml of methanesulfonyl chloride and 0.28 ml of triethylamine. After dropwise, the reaction mixture was stirred for 15 minutes at the same temperature. After reaction, chloroform was added to the reaction mixture and the organic layer was separated, then washed with water and saturated sodium chloride solution. The resulting solution was dried and removed under reduced pressure and the residue was dissolved in 30 ml of acetone. To this solution was added 450 mg of potassium thioacetate at room temperature and stirred for 18 hours at the same temperature. After reaction, ethyl acetate was added to the reaction mixture and the organic layer was separated. The resulting organic solution was washed with water and saturated sodium chloride solution and removed under reduced pressure. The residue was purified by silica gel column chromatography (n-hexane:ethyl acetate = 1:1) to give 810 mg (80 %) of Compound (1) as yellow oil.
MNR ($CDCl_3$) $\delta$ : 2.33 (3H, s), 3.10 (2H, m), 3.20 (3H, m), 3.75 (2H, m), 5.25 (4H, m), 7.30-7.60 (4H, m), 8.00-8.30 (4H, m).

Example 2:

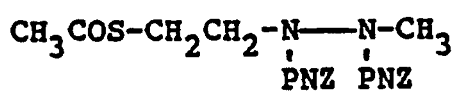

Compound (1)

(a)

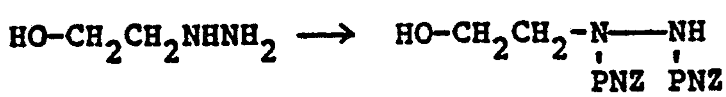

Compound (5)

To an ice-cooling solution of 4.3 g of p-nitrobenzylchloroformate in 10 ml of dioxane were added 1.66 g of sodium bicarbonate, 1 ml of water and a mixture of 760 mg of hydroxyethylhydrazine in 2 ml of water respectively, and the reaction mixture was stirred for 30 minutes at the same temperature. After reaction, ethyl acetate was added to the reaction mixture and the organic layer was washed with water and saturated sodium chloride solution, and dried over magnesium sulfate. The organic solvent was removed under reduced pressure to give 4.4 g of Compound (5) as pale brownish oil.
MNR ($CDCl_3$) $\delta$ : 3.73 (4H, s), 5.27 (4H, s), 7.45 (4H, m), 8.10 (4H, m).

(b)

Compound (5)

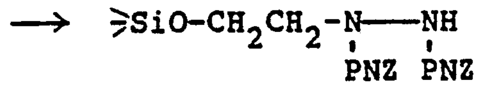

Compound (6)

To a solution of 3.48 g of Compound (5) obtained in the step (a) above in 20 ml of tetrahydrofuran were added dropwise 1.26 ml of chlorotrimethylsilane and 0.86 ml of pyridine under ice-cooling, then the reaction mixture was stirred for 20 minutes at the same temperature. After reaction, ethyl acetate was added to the reaction mixture and the separated organic layer was washed with 5 % of sodium bicarbonate solution, water and saturated sodium chloride solution, and dried over magnesium sulfate. The organic solvent was removed under reduced pressure to give the Compound (6). This compound was used for the next step without further purification.

(c)

Compound (6)

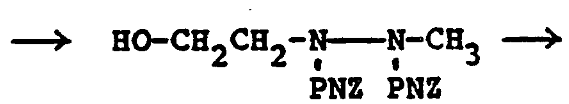

Compound (1)

Compound (4)

To a solution of Compound (6) obtained in the step (b) above in 25 ml of tetrahydrofuran was added 350 mg of sodium hydride (55 %) under ice-cooling, and the reaction mixture was stirred for 30 minutes at the same temperature and then for 30 minutes at room temperature. Then, 1 ml of methyl iodide was added dropwise to the reaction mixture, and the mixture was stirred for 1 hour. After adding 40 ml of 1N-hydrogen chloride solution, the mixture was continued stirring for further 30 minutes. After reaction, ethyl acetate was added to the reaction mixture and the separated organic layer was washed with water and saturated sodium chloride solution. The resulting solvent was dried over magnesium sulfate and removed under reduced pressure to give the residue. This residue was then purified by silica gel column chromatography (n-hexane:ethyl acetate = 1:1) to give 2.5 g of Compound (4) as pale yellow oil.

This Compound (4) was converted to Compound (1) using the same manner as described in the step (d) of Example 1.

Example 3:

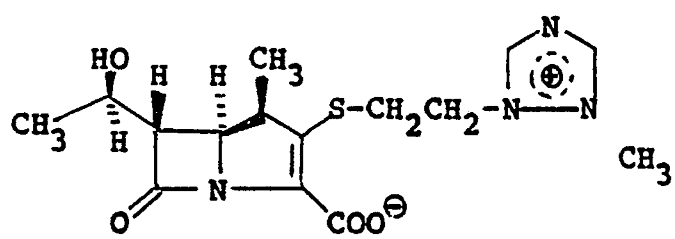

Compound (11)

[A]

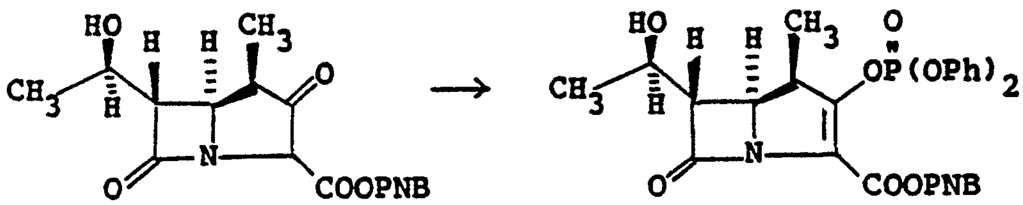

Compound (7) Compound (8)

The Compound (7) as a starting compound in this example was prepared by the process described in the specification of Japanese Laid-Open Patent Publication No. 170,377/1988.

To a solution of 465 mg of Compound (7) in 5 ml of anhydrous acetonitrile were added 0.28 ml of diphenylphosphoric chloride and 0.23 ml of diisopropylethylamine under ice-cooling, and the mixture was stirred for 30 minutes at the same temperature. After the reaction mixture was concentrated, the residue was purified by silica gel column chromatography to give 630 mg of Compound (8) as a white solid.

MNR ($CDCl_3$) δ : 1.24 (3H, d), 1.34 (3H, d), 3.30 (1H, q), 3.52 (1H, m), 4.10-4.40 (2H, m), 5.20 and 5.35 (2H, q), 7.29 (10H, m), 7.58 and 8.18 (4H, d).

[B]

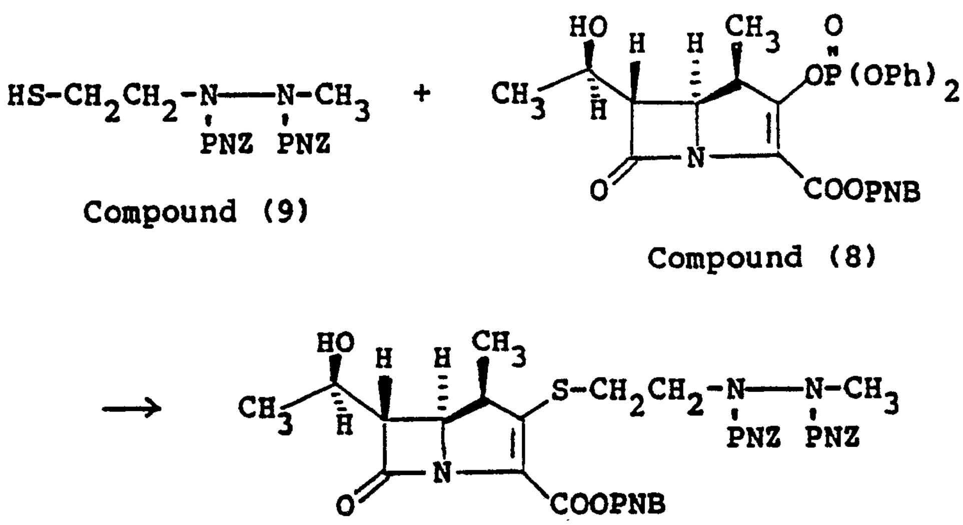

Compound (9)

Compound (8)

Compound (10)

To a solution of 532 mg of Compound (1) obtained in Example 1 or 2 in 10 ml of methanol and 3 ml of tetrahydrofuran was added 0.53 ml of 2N-sodium methoxide in methanol solution under ice-cooling, and the mixture was stirred for 5 minutes at the same temperature. After reaction, chloroform was added to the reaction mixture and the separated organic layer was washed with 1N-hydrogen chloride solution and dried over magnesium sulfate. The resulting solvent was removed under reduced pressure to give Compound (9). This compound was used for next reaction without purification.

To a mixture of Compound (9) obtained above and 594 mg of Compound (8) in 15 ml of acetonitrile was added dropwise 0.18 ml of diisopropylethylamine under ice-cooling, and the reaction mixture was stirred for 1 hour at the same temperature and further 1 hour at room temperature. After the reaction mixture was concentrated, the resulting residue was purified by silica gel column chromatography (chloroform:acetone = 1:1) to give 515 mg of Compound (10) as yellowish solid.

MNR ($CDCl_3$) δ : 1.25 (3H, d, J=8.0Hz), 1.37 (3H, d, J=6.0Hz), 3.00-4.40 (11H, m), 5.10-5.60 (6H, m), 7.30-7.75 (6H, m), 8.05-8.30 (6H, m).

[C]

Compound (10)

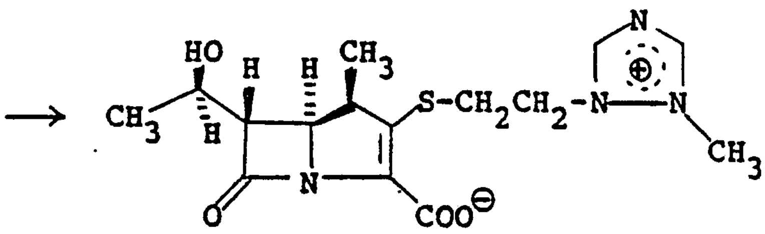

Compound (11)

To a solution of 515 mg of Compound (10) in 8 ml of tetrahydrofuran and 8 ml of 0.1M phosphate buffer (pH = 7.0) were added 107 mg of sodium bicarbonate and 130 mg of 10 % palladium-carbon, and the catalytic hydrogeneration was carried out at room temperature for 2 hours under a pressure of 4.0 atmospheres. After removal of the catalyst and the catalyst was washed with 10 ml of a mixture solution of tetrahydrofuran-water (1:1). The solvent was combined and to this solvent was added 15 ml of n-butanol, then tetrahydrofuran was removed off under reduced pressure. The resulting residue was adjusted to pH 7.0 and to this solution were added 107 mg of sodium bicarbonate and 500 ml of ethylformimidate hydrchloride. After stirred for 15 minutes under ice-cooling, and pH of the reaction mixture was adjusted to 6.0 and the solvent was concentrated under reduced pressure. The resulting residue was purified using HP-40 column (3 % acetone-water) to give 120 mg (53 %) of (1R,5S,6S)-6-[(R)-1-hydroxyethyl]-1-methyl-2-[2-(1-methyl-1,2,4-triazolium-2-yl)ethyl]thio-carbapenem-3-carboxylate [Compound (11)] after lyophilization.

100 mg of this lyophilized Compound (11) was dissolved in 50µl of water, and to this solution were added 450 µl of ethanol and 100 µl of acetone and the mixture was stirred for 30 minutes under room temperature. After stirring, the resulting crystals were collected and washed with a small amount of a mixture solvent of water:ethanol:acetone (= 1:9:2) and the resultant crystals were dried under a vacuum to give 63 mg of crystalline Compound (11).
MNR ($CDCl_3$) δ: 1.20 (3H, d, J = 7.0Hz), 1.27 (3H, d, J = 6.0Hz), 3.00-4.30 (8H, m), 4.25 (3H, s), 9.07 (1H, s), 9.27 (1H, s).

The carbapenem compounds according to the present invention may be formulated in various preparation forms.

Formulation Example 1 (Injection):

| (1) Injectable suspension: | |
|---|---|
| Compound (11) | 0.25 g |
| Methyl cellulose | 0.5 g |
| Polyvinyl pyrrolidone | 0.05 g |
| Methyl p-oxybenzoate | 0.1 g |
| Polysolvate 80 | 0.1 g |
| Lidocaine hydrochloride | 0.5 g |
| Distilled water | to make 100 ml |

The above components were formulated into 100 ml of an injectable suspension.

(2) Lyophilization:

An appropriate amount of distilled water was added to 20 g of Compound (11) to make a total volume of 100 ml. The above solution (2.5 ml) was filled in vials so as for each vial to contain 500 mg of Compound (11) and lyophilized. The lyophilized vial was mixed in situ with approximately 3-4 ml of distilled water to

make an injectable solution.

(3) Powder:

Compound (11) was filled in an amount of 250 ml in a vial and mixed in situ with about 3-4 ml of distilled water to make an injectable solution.

| Formulation Example 2 (Tablets): | |
|---|---|
| Compound (11) | 250 mg |
| Lactose | 250 mg |
| Hydroxypropyl cellulose | 1 mg |
| Magnesium stearate | 10 mg |
| | 511 mg/tablet |

The above components were mixed with each other and punched into tablets in conventional manner. Such tablets, as required, may be formulated into sugar coatings or film coatings in conventional manner.

| Formulation Example 3 (Troche): | |
|---|---|
| Compound (11) | 200 mg |
| Sugar | 770 mg |
| Hydroxypropyl cellulose | 5 mg |
| Magnesium stearate | 20 mg |
| Flavor | 5 mg |
| | 1,000 mg/troche |

The component was mixed with each other and formulated into troches by punching in conventional manner.

| Formulation Example 4 (Capsules): | |
|---|---|
| Compound (11) | 500 mg |
| Magnesium stearate | 10 mg |
| | 510 mg/capsule |

The component was mixed with each other and filled in conventional hard gelatin capsules.

| Formulation Example 5 (Dry Syrup): | |
|---|---|
| Compound (11) | 200 mg |
| Hydroxypropyl cellulose | 2 mg |
| Sugar | 793 mg |
| Flavor | 5 mg |
| | 1,000 mg |

The above components were mixed with each other and formulated into dry syrups in conventional manner.

| Formulation Example 6 (Powders): | | |
|---|---|---|
| (1) | Compound (11)<br>Lactose | 200 mg<br>800 mg<br>1,000 mg |
| (2) | Compound (11)<br>Lactose | 250 mg<br>750 mg<br>1,000 mg |

Each of the components was mixed with each other and formulated in powders in conventional manner.

| Formulation Example 7 (Suppository): | |
|---|---|
| Compound (11)<br>Witepsol H-12 (Product of Dynamite Noble) | 500 mg<br>700 mg<br>1,200 mg |

The above components were mixed with each other and formulated into suppositories in conventional manner.

## Claims

1. A (1R,5S,6S)-6-[(R)-1-hydroxyethyl]-1-methyl-2-[(1-alkyl-1,2,4-triazolium-2-yl)alkyl]thio-carbapenem-3-carboxylate represented by the following formula

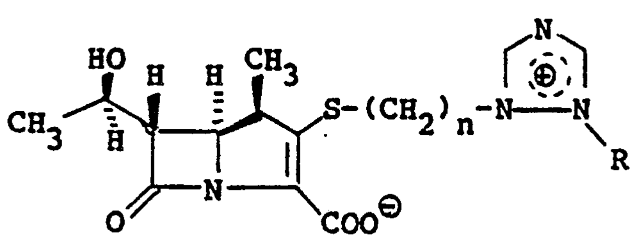

(I)

wherein R is a lower alkyl group, and n is 1, 2 or 3, or a pharmaceutically acceptable salt thereof.

2. The compound of Claim 1 which is (1R,5S,6S)-6-[(R)-1-hydroxyethyl]-1-methyl-2-[2-(1-methyl-1,2,4-triazolium-2-yl)ethyl]thio-carbapenem-3-carboxylate of the following formula

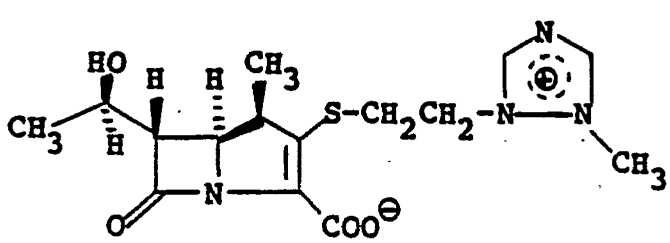

(I-1)

or a pharmaceutically acceptable salt thereof.

3. The compound of Claim 2 which is the compound of formula (I-1) in a crystalline form, or a pharmaceutically acceptable salt thereof.

4. The compound of Claim 1 as an antibacterial agent.

5. (1R,5S,6S)-6-[(R)-1-hydroxyethyl]-1-methyl-2-[2-(1-methyl-1,2,4-triazolium-2-yl)ethyl]thio-carbapenem-3-carboxylate or a pharmaceutically acceptable salt thereof as an antibacterial agent.

6. A process for producing a (1R,5S,6S)-6-[(R)-1-hydroxyethyl)-1-methyl-2-[(1-alkyl-1,2,4- triazolium-2-yl)alkyl]thio-carbapenem-3-carboxylate represented by the following formula:

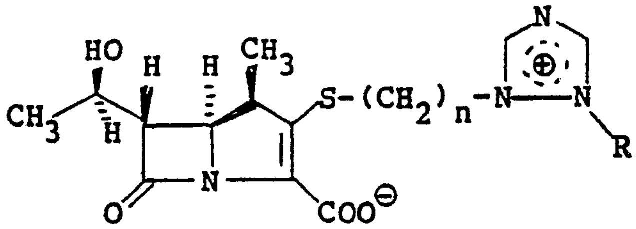

(I)

wherein R is a lower alkyl group, and n is 1, 2 or 3,
or a pharmaceutically acceptable salt thereof;
which comprises reacting a compound represented by the following formula (II):

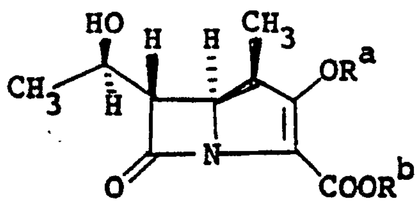

(II)

wherein $R^a$ is an acyl group, and $R^b$ is a carboxyl protecting group,
with a mercapto reagent represented by formula (III):

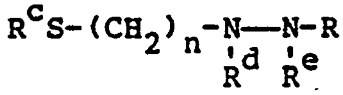

(III)

wherein $R^c$ is a hydrogen atom or an acyl group; $R^d$ and $R^e$ are amino protecting groups; and R and n have the same meanings as above,
to give a compound represented by formula (IV):

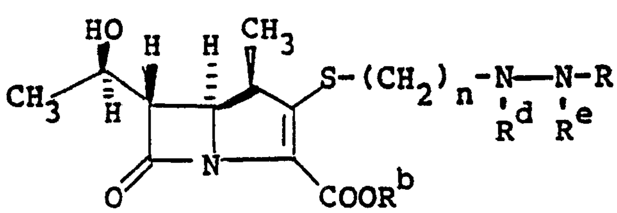

(IV)

wherein R, $R^b$, $R^d$, $R^e$ and n have the same meanings as above,
and subjecting the compound of the formula (IV) to removal of the amino protecting groups $R^d$ and Re, and of the carboxyl protecting group $R^b$ respectively, and then reacting with formimidic acid ester to give the carbapenem compound represented by formula (I).

7. The process of Claim 6 for producing a (1R,5S,6S)-6-[(R)-1-hydroxyethyl]-1-methyl-2-[2-(1-methyl-1,2,4-triazolium-2-yl)ethyl]thio-carbapenem-3-carboxylate (I-1).

8. An antibacterial agent comprising a compound of formula (I) defined in Claim 1 of a pharmaceutically acceptable salt thereof.

9. An antibacterial agent comprising (1R,5S,6S)-6-[(R)-1-hydroxyethyl]-1-methyl-2-[2-(1-methyl-1,2,4-triazolium-2-yl)ethyl]thio-carbapenem-3-carboxylate or a pharmaceutically acceptable salt thereof.

10. A use of the compound according to Claim 1 for controlling or preventing a bacterial infection.

11. A use of (1R,5S,6S)-6-[(R)-1-hydroxyethyl]-1-methyl-2-[2-(1-methyl-1,2,4-triazolium-2-yl)ethyl]thio-carbapenem-3-carboxylate or a pharmaceutically acceptable salt thereof for controlling or preventing a

bacterial infection.

12. A use of the compound according to claim 1 for the manufacture of antibacterial medicaments.

13. A use of (1R,5S,6S)-6-[(R)-1-hydroxyethyl]-1-methyl-2-[2-(1-methyl-1,2,4-triazolium-2-yl)ethyl]thio-carbapenem-3-carboxylate or a pharmaceutically acceptable salt thereof for the manufacture of antibacterial medicaments.

Claims for the following Contracting State: GR

1. A (1R,5S,6S)-6-[(R)-1-hydroxyethyl]-1-methyl-2-[(1-alkyl-1,2,4-triazolium-2-yl)alkyl]thio-carbapenem-3-carboxylate represented by the following formula

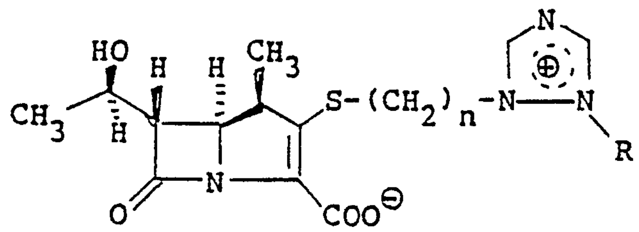

(I)

wherein R is a lower alkyl group, and n is 1, 2 or 3,
or a salt thereof.

2. The compound of Claim 1 which is (1R,5S,6S)-6-[(R)-1-hydroxyethyl]-1-methyl-2-[2-(1-methyl-1,2,4-triazolium-2-yl)ethyl]thio-carbapenem-3-carboxylate of the following formula

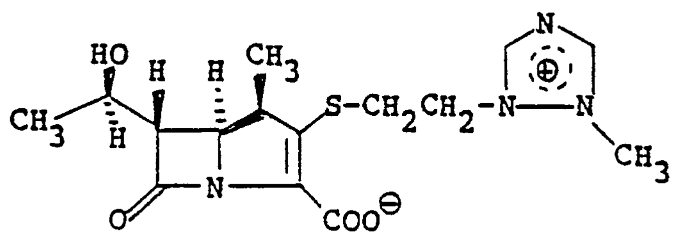

(I-1)

or a salt thereof.

3. The compound of Claim 2 which is the compound of formula (I-1) in a crystalline form, or a salt thereof.

4. A process for producing a (1R,5S,6S)-6-[(1R)-1-hydroxyethyl]-1-methyl-2-[(1-alkyl-1,2,4- triazolium-2-yl)alkyl]thio-carbapenem-3-carboxylate represented by the following formula:

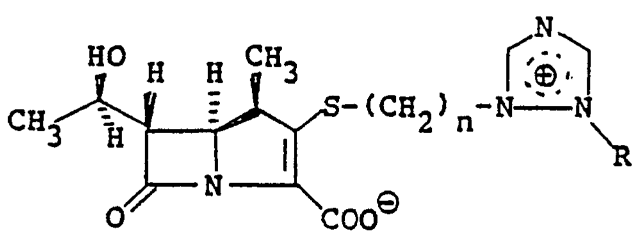

(I)

wherein R is a lower alkyl group, and n is 1, 2 or 3,
or a salt thereof;
which comprises reacting a compound represented by the following formula (II):

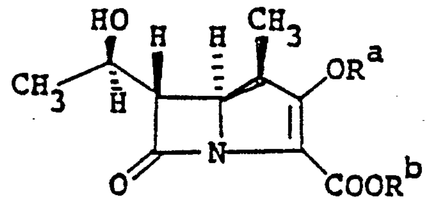

(II)

wherein $R^a$ is an acyl group, and $R^b$ is a carboxyl protecting group, with a mercapto reagent represented by formula (III):

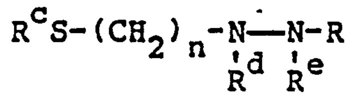

(III)

wherein $R^c$ is a hydrogen atom or an acyl group; $R^d$ and $R^e$ are amino protecting groups; and R and n have the same meanings as above,
to give a compound represented by formula (IV):

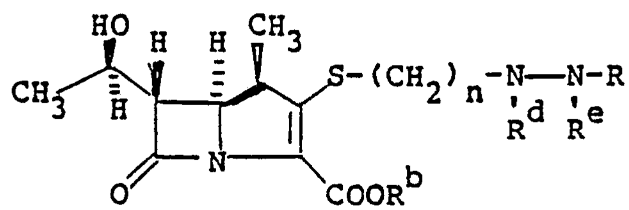

(IV)

wherein R, $R^b$, $R^d$, $R^e$ and n have the same meanings as above,
and subjecting the compound of the formula (IV) to removal of the amino protecting groups $R^d$ and $R^e$, and of the carboxyl protecting group $R^b$ respectively, and then reacting with formimidic acid ester to give the carbapenem compound represented by formula (I).

5. The process of Claim 4 for producing a (1R,5S,6S)-6-[(R)-1-hydroxyethyl]-1-methyl-2-[2-(1-methyl-1,2,4-triazolium-2-yl)ethyl]thio-carbapenem-3-carboxylate (I-1).

Claims for the following Contracting State: ES

1. A process for producing a (1R,5S,6S)-6-[(R)-1-hydroxyethyl]-1-methyl-2-[(1-alkyl-1,2,4-triazolium-2-yl)alkyl]thio-carbapenem-3-carboxylate represented by the following formula:

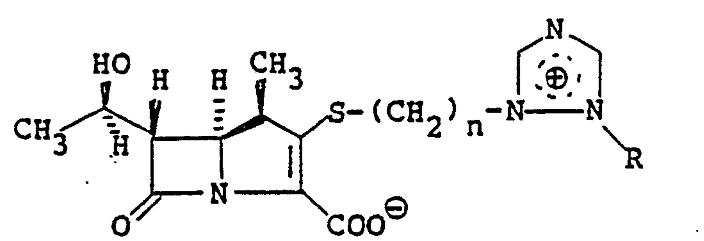

(I)

wherein R is a lower alkyl group, and n is 1, 2 or 3,
or a pharmaceutically acceptable salt thereof;
. which comprises reacting a compound represented by the following formula (II):

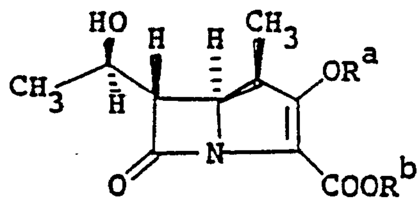

(II)

wherein $R^a$ is an acyl group, and $R^b$ is a carboxyl protecting group, with a mercapto reagent represented by formula (III):

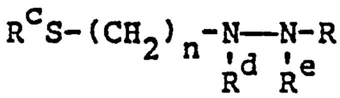

(III)

wherein $R^c$ is a hydrogen atom or an acyl group; $R^d$ and $R^e$ are amino protecting groups; and R and n have the same meanings as above,
to give a compound represented by formula (IV):

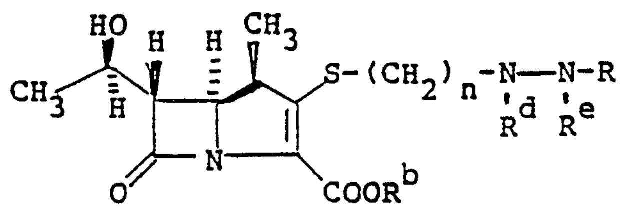

(IV)

wherein R, $R^b$, $R^d$, $R^e$ and n have the same meanings as above,
and subjecting the compound of the formula (IV) to removal of the amino protecting groups $R^d$ and $R^e$, and of the carboxyl protecting group $R^b$ respectively, and then reacting with formimidic acid ester to give the carbapenem compound represented by formula (I). .

2. The process of Claim 1 for producing a (1R,5S,6S)-6-[(R)-1-hydroxyethyl]-1-methyl-2-[2-(1-methyl-1,2,4-triazolium-2-yl)ethyl]thio-carbapenem-3-carboxylate (I-1).

European Patent Office

# EUROPEAN SEARCH REPORT

Application Number

EP 89 11 5883

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 168 707 (MERCK)<br>* Claims; especially page 167, compounds 92,93 * | 1-13 | C 07 D 477/00<br>A 61 K 31/40 |
| Y | EP-A-0 170 073 (MERCK)<br>* Pages 56-60, example 10; page 86, example 167; claims * | 1-13 | |
| Y | EP-A-0 169 410 (MERCK)<br>* Pages 102-103; claims * | 1-13 | |
| P,Y | PATENT ABSTRACTS OF JAPAN, vol. 13, no. 63 (C-568)[3411], 13th February 1989; & JP-A-63 255 283 (NIPPON REDARII K.K.) 21-10-1988<br>* Abstract * | 1-13 | |

TECHNICAL FIELDS SEARCHED (Int. Cl.5)

C 07 D 487/00
C 07 D 249/00
C 07 D 233/00
A 61 K 31/00

The present search report has been drawn up for all claims

| Place of search | Date of completion of the search | Examiner |
|---|---|---|
| THE HAGUE | 24-11-1989 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)